# EUROPEAN PATENT SPECIFICATION

(11) **EP 1 696 866 B1**
(45) Date of publication and mention of the grant of the patent: **23.10.2013**
(21) Application number: 04815090.8
(22) Date of filing: 17.12.2004
(51) Int. Cl.: A61K 8/22, A61Q 11/00, A61K 8/19

(54) **Emulsion composition for delivery of bleaching agents to teeth**
Emulsionszusammensetzung zur Abgabe eines Bleichmittels an Zähne
Composition d'émulsion destinée à appliquer sur les dents des agents de blanchîment

(30) Priority: 17.12.2003 US 530397 P; 17.12.2003 US 530387 P; 17.12.2003 US 530217 P
(43) Date of publication of application: 06.09.2006
(73) Proprietor: The Procter & Gamble Company, Cincinnati, OH 45202 (US)
(72) Inventor: QUAN, Ke Ming, West Chester, Ohio 45069 (US); PEGOLI, Ronald Edward, Loveland, Ohio 45140 (US); GHOSH, Chanchal Kumar, West Chester, o 45069 (US)
(74) Representative: Joos, Uta Susanne
(86) International application number: PCT/US2004/042973
(87) International publication number: WO 2005/058267

(56) References cited:
- EP-A- 1 312 354
- US-A- 4 606 913
- US-A1- 2002 176 827
- US-A1- 2002 187 108
- US-A1- 2003 180 229

## Description

The present invention relates to a composition for whitening teeth comprising an emulsion. The present compositions comprise a bleaching agent, an emulsifier, an aqueous phase and an inert hydrophobic phase, wherein the hydrophobic (e.g. oil) phase is in predominant proportion relative to the aqueous phase. In one embodiment the invention relates to a delivery system comprising the present compositions and an integral carrier, e.g. a strip of material, a dental tray, and/or a sponge material.

### Background of the Invention

Dental products by which various cosmetic and/or therapeutic actives are delivered to teeth and the oral cavity are known. Examples of such products include: brushing aids such as dentifrice products for delivery of oral care actives such as polyphosphates or fluorides; mouthwashes containing breath fresheners or antibacterial actives; and whitening strips for the delivery of bleaching actives to the teeth. In particular the use of a dental strip has been recognized as a convenient and inexpensive way to deliver cosmetic and therapeutic benefits to the teeth and mucosal surfaces of the oral cavity. For example, dental whitening strips, where a whitening composition is applied to a strip and thereafter applied to the teeth to achieve sustained contact between the teeth and the whitening composition, are known. See U.S. Pat. Nos. 6,136,297; 6,096,328; 5,894,017; 5,891,453; and 5,879,691, all to Sagel, et al., and U.S. Pat. Nos. 5,989,569 and 6,045,811 both to Dirksing, et al., all assigned to The Procter & Gamble Company. US 2002/0187108 discloses oral compositions, including bleaching compositions, comprising polybutene. US 2002/0176827 describes similar compositions applied to a strip for use in the mouth.

Despite the above Known approaches for the treatment of oral conditions, especially for the whitening of teeth, a need still exists for providing products with both improved bleaching efficacy as well as increased speed of whitening. The prior art has generally attempted to address these concerns by increasing the level of the bleaching agent in the compositions. This approach, however, presents several problems. First the subject may experience increased irritation and/or sensitivity which may be associated with using an increased amount of a bleaching agent. Furthermore, some regulatory authorities and legislation in various geographies throughout the world do not allow bleaching agents to be used in products at levels above certain concentrations. Therefore, despite the above known approaches for the treatment of oral conditions, especially for the whitening of teeth, a need still exists for providing products with improved bleaching efficacy. The present invention overcomes some of the limitations of the prior art, and relates to a composition comprising a bleaching agent, an emulsifier, an aqueous phase and an inert hydrophobic phase, wherein the inert hydrophobic phase is in predominant proportion relative to the aqueous phase.

### SUMMARY OF THE INVENTION

The present invention relates to a bleaching composition, in the form of a water-in-oil emulsion, for whitening teeth comprising:
a. from 1% to 45%, by weight, of an aqueous phase;
b. a safe and effective amount of a bleaching agent;
c. at least 30%, by weight, of an inert, continuous hydrophobic phase; and
d. from 0.001% to 30%, by weight, of an emulsifier;
wherein the composition is an emulsion in which the inert hydrophobic phase is in predominant proportion relative to the aqueous phase.

In one embodiment an oral care delivery system comprises an integral carrier with the present composition coated on the carrier. The present invention is used to deliver whitening benefits to the oral cavity by directly applying the composition or the integral carrier to the teeth. The integral carrier is attached to the teeth via the compositions herein or the adhesion function can be provided independent of the present compositions herein (e.g. can be provided via a separate adhesive composition used with the present compositions and integral carrier). Alternatively, the integral carrier may be attached to the teeth via an attachment means that is part of the integral carrier, for example the integral carrier may optionally be of sufficient size that, once applied, the integral carrier overlaps with the oral soft tissues rendering more of the teeth surface available for bleaching.

### BRIEF DESCRIPTION OF THE DRAWINGS

While the specification concludes with claims that particularly point out and distinctly claim the present invention, it is believed that the present invention will be understood better from the following description of embodiments, taken in conjunction with the accompanying drawings, in which like reference numerals identify identical elements. The integral carrier includes a strip of material, dental tray, sponge material, and mixtures thereof. In one embodiment of the present invention, the integral carrier comprises a strip of material. The strip of material is attached to the teeth via an attachment means. For example, the attachment means may be the present composition, an adhesive composition separate from the present composition, or an attachment means that is part of the integral carrier, e.g. the integral carrier may optionally be of sufficient size and/or width and have sufficient adhesiveness, that, once applied, the integral carrier overlaps with the oral soft tissues rendering more of the teeth surface available for bleaching.

Without intending to limit the invention, the strip of material embodiment is described in more detail below:
FIG. 1 is a perspective view of a substantially flat strip of material having rounded comers;
FIG. 2 is a perspective view of an embodiment of the present invention, disclosing the strip of FIG. 1 upon which a second layer composition comprising the present compositions wherein the bleaching agent is releaseably associated with the integral carrier and/or the present composition;
FIG. 3 is a cross-sectional view, taken along section line 3-3 of FIG. 2, showing an example of the strip of material having a thickness less than that of the second layer coated thereon;
FIG. 4 is a cross-sectional view, showing an alternative embodiment of the present invention, showing shallow pockets in the strip of material, which act as reservoirs for additional amounts of the second layer coated on the strip;
FIG. 5 is a cross-sectional plan view, showing an alternative embodiment for applying the second layer composition to adjacent teeth having the strip of material of the present invention conforming thereto and adhesively attached to the teeth by means of the second layer composition located between the teeth and the strip of material;
FIG. 6 is a cross-sectional elevation view of a tooth, taken along section line 6-6 of FIG. 5, showing the strip of material of the present invention conforming to and adhesively attached to the teeth by means of the second layer composition located between the teeth and strip of material;
FIG. 7 is a cross-sectional plan view, similar to FIG. 5, showing a strip of material of the present invention conforming to the teeth and the adjoining soft tissue and adhesively attached to both sides of the teeth by means of the second layer composition located between the teeth and the strip of material;
FIG. 8 is a cross-sectional elevation view, taken along section line 8-8 of FIG.7, showing a strip of material of the present invention conforming to both the tooth and the adjoining soft tissue and adhesively attached to both sides of the tooth by means of the second layer composition located between the teeth and the strip of material;
FIG. 9 is a perspective view of an alternative embodiment of the present invention, disclosing the strip of material coated with a second layer composition of FIG. 2 for treating teeth and adjoining soft tissue having a release liner;
FIG. 10 is a cross-sectional view of an alternative embodiment of the present invention, taken along section line 10-10 of FIG. 9, showing a release liner attached to the strip of material by the second layer composition on the strip of the material.

### DETAILED DESCRIPTION

### Definitions

By "oral care composition" or "oral composition" as used herein is meant a product which is not intentionally swallowed for purposes of systemic administration of therapeutic agents, but is retained in the oral cavity for a sufficient time to contact the dental surfaces for purposes of whitening efficacy.

By "safe and effective amount" as used herein is meant an amount of a component, high enough to significantly (positively) modify the condition to be treated or to effect the desired whitening result, but low enough to avoid serious side effects (at a reasonable benefit/risk ratio), within the scope of sound medical/dental judgment. The safe and effective amount of a component, will vary with the particular condition (e.g., to effect whitening) being treated, the age and physical condition of the patient being treated, the severity of the condition, the duration of treatment, the nature of concurrent therapy, the specific form employed, and the particular vehicle from which the component is applied.

By "a sufficient period of time to achieve whitening" as used herein means that the composition is used or worn by the subject or the subject is instructed to use or wear the composition for greater than 2 minutes, in another embodiment from about 2.5 minutes to about 12 hours (e.g overnight treatment), in another embodiment from about 3 minutes to about 120 minutes, in yet another embodiment from about 5 minutes to about 40 minutes, per application, and may be applied from about 1 to about 7 times per day. Additionally, the length of treatment to achieve the desired benefit, for example, tooth whitening, may last from about one day to about six months, in another embodiment from about one day to about 28 days, and in another embodiment from about 7 to about 28 days. The optimal duration and frequency of application will depend on the desired effect, the severity of any condition being treated, the health and age of the user and like considerations.

By "cm" as used herein means centimeter. By "mm" as used herein means millimeter.

All percentages and ratios used herein after are by weight of total composition, unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

### Emulsion Compositions

The compositions herein comprise an aqueous phase, an immiscible inert hydrophobic phase, bleaching agent, as well as an emulsifier, resulting in a water in oil emulsion. One phase is the dispersed, internal or discontinuous phase and the other phase is the dispersion medium, external phase or continuous phase. The dispersed phase is generally dispersed throughout the continuous phase as small droplets. The components of the two immiscible phases are chosen to allow for the release of the bleach agent readily from the composition.

Without being bound by theory, when the present invention is brought into contact with a hydrophilic surface such as the tooth surface, the aqueous phase may attempt to separate from the hydrophobic phase towards the hydrophilic tooth surface. The possible net effect is that the tooth whitening efficacy may be enhanced and/or accelerated.

Without being bound by theory, the present invention may increase the "effective" concentration of the bleaching agent on the surface of the teeth. Therefore, increased speed of whitening and increased efficacy of the bleaching agent may be achieved, even though the same or lower total level of the bleaching agent is used. The present invention, therefore, at a given total concentration of bleaching agent, may require fewer applications to get the same degree of whitening or may require a lower gel load to get the same degree of whitening. Also, due to the replacement of some of the aqueous phase with an inert hydrophobic phase, retention of the composition on the tooth surfaces may be improved.

Furthermore, the inert hydrophobic phase may provide a more stable matrix for flavor ingredients, especially those flavor ingredients that are soluble in the inert hydrophobic phase. The inert hydrophobic phase may provide greater hydration of the teeth surfaces.

Lastly, the emulsion may also form a "film" over the soft tissues of the oral cavity such as the gums, to decrease tooth sensitivity that is sometimes associated with bleaching agents applied in the oral cavity.

### Aqueous Phase

The present compositions comprise from 1% to 45% of an aqueous phase comprising water and, optionally water dispersible/miscible liquids. In one embodiment the level of the aqueous phase is from 1% to 35%, in another embodiment from 1% to 25%, in another embodiment from 1% to 20%, by weight of the composition. In another embodiment very low levels of aqueous phase may be useful, for example, from 1% to 10%, in another embodiment from 2% to 8%, in another embodiment from 2% to 5%, by weight of the composition.

The aqueous phase is generally selected from the group consisting of water, polyalkylene glycols with molecular weights from 200 to 20,000, humectants, and mixtures thereof. Humectants generally include edible polyhydric alcohols such as glycerin, sorbitol, xylitol, butylene glycol, polyethylene glycol, and propylene glycol, and mixtures thereof. In one embodiment the aqueous phase comprises only water. In one embodiment the aqueous phase comprises at least 10% water, in another embodiment at least 20% by weight of the aqueous phase, of water.

### Bleaching Agent

The present compositions further comprise a safe and effective amount of a bleaching agent. In one embodiment the level of bleaching agent is dependent on the available oxygen or chlorine respectively that the molecule is capable of providing to bleach the stain. In one embodiment the bleaching agent level is from 0.1% to 20%, in another embodiment from 0.5 to 9% and in another embodiment from 3% to 8% by weight of the composition, of the bleaching agent. In one embodiment the bleaching agent is surprisingly more effective when used at lower levels, generally from 0.5% to 3%, in another embodiment from 0.5% to 1.5% by weight of the composition.

In one embodiment the bleaching agents are selected from the group consisting of the peroxides, metal chlorites, perborates, percarbonates, peroxyacids, persulfates, compounds that form the preceding compounds in situ, and combinations thereof. Suitable peroxide compounds include hydrogen peroxide, urea peroxide, calcium peroxide, carbamide peroxide, and mixtures thereof. In one embodiment the bleaching agent is carbamide peroxide. Suitable metal chlorites include calcium chlorite, barium chlorite, magnesium chlorite, lithium chlorite, sodium chlorite, potassium chlorite, and mixtures thereof. Additional bleach agents also include hypochlorite and chlorine dioxide. In one embodiment the bleaching agent is selected from the group consisting of sodium chlorite, sodium percarbonate, oxones, and mixtures thereof. The starting bleach material can be aqueous or solid material.

### Inert Hydrophobic Phase

The present compositions comprise at least 30% of an inert, continuous, hydrophobic phase. The inert hydrophobic phase is a hydrophobic material, which is water insoluble or water immisible, non-toxic to the user and chemically stable and compatible with other ingredients present in the composition. In one embodiment the present composition comprises from 40% to 90% and in another embodiment from 50% to 85%, and in another embodiment from 65% to 80% by weight of the composition, of the inert hydrophobic phase as the continuous phase. The inert hydrophobic phase is in predominant proportion relative to the aqueous phase present in the composition. As used herein "predominant proportion" means that the percent by weight of the composition of the inert hydrophobic phase is in excess relative to the percent by weight of the composition of the aqueous phase. In one embodiment the inert hydrophobic phase is a non-toxic oil.

In one embodiment the inert hydrophobic phase is selected from the group consisting of non-toxic edible oils, saturated or unsaturated fatty alcohols, saturated aliphatic hydrocarbons, long chain triglycerides, fatty esters, and mixtures thereof. In one embodiment the saturated aliphatic hydrocarbons contain from 10 to 20 carbon atoms such as decane, 2 ethyldecane, tetradecane, isotetradecane, hexadecane, eicosane, and mixtures thereof. In another embodiment the inert hydrophobic phase is selected from the group consisting of silicones, polysiloxanes, and mixtures thereof.

Long chain triglycerides include vegetable oils, fish oils, animal fats, hydrogenated vegetable oils, partially hydrogenated vegetable oils, semi-synthetic triglycerides, synthetic triglycerides, and mixtures thereof. Fractionated, refined or purified oils of these types can also be used.

Specific examples of suitable long chain triglyceride-containing oils suitable for use in the compositions of the present invention include almond oil; babassu oil; borage oil; black currant seed oil; canola oil; castor oil; coconut oil; corn oil; cottonseed oil; emu oil; evening primrose oil; flax seed oil; grapeseed oil; groundnut oil; mustard seed oil; olive oil; palm oil; palm kernel oil; peanut oil; rapeseed oil; safflower oil; sesame oil; shark liver oil; soybean oil; sunflower oil; hydrogenated castor oil; hydrogenated coconut oil; hydrogenated palm oil; hydrogenated soybean oil; hydrogenated vegetable oil; a mixture of hydrogenated cottonseed oil and hydrogenated castor oil; partially hydrogenated soybean oil; a mixture of partially hydrogenated soybean oil and partially hydrogenated cottonseed oil; glyceryl trioleate; glyceryl trilinoleate; glyceryl trilinolenate; a Ω3 polyunsaturated fatty acid triglyceride containing oil; and mixtures thereof.

In one embodiment long chain triglyceride containing oils are selected from the group consisting of corn oil; olive oil; palm oil; peanut oil; safflower oil; sesame oil; soybean oil; hydrogenated castor oil; partially hydrogenated soybean oil; glyceryl trioleate; glyceryl trilinoleate; a Ω3 polyunsaturated fatty acid triglyceride containing oil; and mixtures thereof.

In one embodiment suitable saturated or unsaturated fatty alcohols have from about 6 to about 20 carbon atoms, cetearyl alcohol, lauryl alcohol, and mixtures thereof. For example, Lipowax (Cetearyl Alcohol and Ceteareth-20) are supplied and manufactured by Lipo Chemical. In one embodiment the fatty alcohols are saturated fatty alcohols have from 6 to 20 carbon atoms.

In one embodiment the inert hydrophobic phase is selected from the group consisting of mineral oil, castor oil, linseed oil, rape oil, peanut oil, soybean oil, rice bran oil, coconut oil, palm oil, safflower oil, olive oil, vegetable oils, corn oil; sesame oil; hydrogenated castor oil; partially hydrogenated soybean oil; glyceryl trioleate; glyceryl trilinoleate; a Ω3 polyunsaturated fatty acid triglyceride containing oil; and mixtures thereof. In another embodiment the inert hydrophobic phase is mineral oil.

General information on silicones including silicone fluids, gums and resins, as well as the manufacture of silicones, can be found in Encyclopedia of Polymer Science and Engineering, Volume 15, Second Edition, pp 204-308, John Wiley & Sons Inc. 1989 and Chemistry and Technology of Silicones, Walter Noll, Academic Press Inc, (Harcourt Brue Javanovich, Publishers, New York), 1968, pp 282-287 and 409-426.

### Emulsifier

The present compositions comprise from 0.001% to 30% of an emulsifier. Any emulsifier may be used as long as the emulsifier chosen is non-toxic to the user, chemically stable and compatible with other ingredients present in the composition, especially the bleach. In one embodiment the emulsifier (or a combination of emulsifiers) favors the formation of a water in oil emulsion. In one embodiment the present compositions comprise from from 0.1 to 5%, in another embodiment from 0.1 to 3%, and in another embodiment from 0.5% to 1.5% by weight of the composition, of emulsifier.

Classes of surfactants useful as emulsifiers include nonionic, anionic, cationic, amphoteric, synthetic emulsifying agents, and mixtures thereof. Many suitable nonionic and amphoteric surfactants are disclosed by U.S. Pat. Nos. 3,988,433 to Benedict; U.S. Patent 4,051,234, issued September 27, 1977, and many suitable nonionic surfactants are disclosed by Agricola et al., U.S. Patent 3,959,458, issued May 25, 1976.

In one embodiment, since water in oil emulsion are favored with more lipophilic emulsifiers, the emulsifier herein has an HLB value of from 1 to 10, in another embodiment the emulsifier has an HLB value of from 3 to 8, and in another embodiment from 4 to 7, and in yet another embodiment from 4 to 6. Either a single emulsifier is used or a combination of emulsifiers are used. In one embodiment a combination of emulsifiers is used wherein the weighted HLB is from 1 to 10, in another embodiment from 3 to 8, and in another embodiment from 4 to 7, and in yet another embodiment from 4 to 6. In another embodiment the emulsifier is a blend of two or more emulsifiers such as a blend of two or more nonionic emulsifiers. In this regard an emulsifer that tends to form a water in oil emulsion and an emulsifier that forms an oil in water emulsion may be blended to achieve the requisite HLB for a water in oil emulsion. (HLB values are algebraically additive.)

Emulsifiers also useful herein include natural emulsifying agents such as acacia, gelatin, lecithin and cholesterol; fmely dispersed solids such as colloidal clays, bentonite, veegum (magnesium aluminum silicate; and synthetic emulsifying agents such as salts of fatty acids, sulfates such as sorbitan trioleate, sorbitan tristearate, sucrose distearate, propylene glycol monostearate, glycerol monostearate, propylene glycol monolaurate, sorbitan monostearate, sorbitan monolaurate, polyoxyethylene-4-lauryl ether, sodium lauryl sulfate, sulfonates such as dioctyl sosium sulfosuccinate, glyceryl esters, polyoxyethylene glycol esters and ethers, diethylene glycol monostearate, PEG 200 distearate, and sorbitan fatty acid esters, such as sorbitan monopalmitate, and their polyoxyethylene derivatives, polyoxyethylene glycol esters such as the monostearate, Polysorbate 80 (ethoxylated sorbitan monooleate) (supplied by Spectrum, etc.), and mixtures thereof.

In one embodiment the emulsifier is a surfactant that is non reactive with the bleach agent. For example, surfactants that are non-reactive with the bleach agent have no hydroxy groups, are free of nitrogen groups and linkages, are essentially free of metals such as Zn, etc.

In one embodiment the emulsifier is a non-ionic surfactant. Nonionic surfactants include polyoxyethylene sorbitan fatty acid esters, e.g., materials sold under the trademark Tween. Examples of such materials are polyoxyethylene (20) sorbitan monolaurate (Tween 20), polyoxyethylene (20) sorbitan monopalmitate (Tween 40), polyoxyethylene (20) sorbitan monostearate (Tween 60), polyoxyethylene (4) sorbitan monostearate (Tween 61), polyoxyethylene (20) sorbitan tristearate (Tween 65), polyoxyethylene (20) sorbitan monooleate (Tween 80), polyoxyethylene (5) sorbitan monooleate (Tween 81), and polyoxyethlene (20) sorbitan trioleate (Tween 85), and mixtures thereof. Polyoxyethylene fatty acid esters are also suitable and examples include those materials sold under the trademark Myrj such as polyoxyethylene (8) stearate (Myrj 45) and polyoxyethylene (40) stearate (Myrj 52), and mixtures thereof. Further nonionics include polyoxyethylene polyoxypropylene block polymers such as poloxamers and Pluronics.

Another suitable class of non-ionic surfactants for use in the present invention are polyoxyethylene fatty ethers, e.g., the materials sold under the trademark Brij. Examples of such materials are polyoxyethylene (4) lauryl ether (Brij 30), polyoxyethylene (23) lauryl ether (Brij 35), polyoxyethylene (2) cetyl ether (Brij 52), polyoxyethylene (10) cetyl ether (Brij 56), polyoxyethylene (20) cetyl ether (Brij 58), polyoxyethylene (2) stearyl ether (Brij 72), polyoxyethylene (10) stearyl ether (Brij 76), polyoxyethylene (20) stearyl ether (Brij 78), polyoxyethylne (2) oleyl ether (Brij 93), polyoxyethylene (10) oleyl ether, and polyoxyethylene (20) oleyl ether (Brij 99), and mixtures thereof.

In one embodiment of the invention, a portion of a non-ionic surfactant employed in the composition of the invention can be substituted with a lipophilic surfactant, e.g., sorbitan fatty acid esters such as the materials sold under the trademark Arlacel. Suitable lipophilic surfactants include sorbitan monolaurate (Arlacel 20), sorbitan monopalmitate (Arlacel 40), sorbitan monostearate (Aracel 60), sorbitan monooleate (Arlacel 80), sorbitan sesquioleate (Arlacel 83), and sorbitan trioleate (Arlacel 85), and mixtures thereof. Typically, from about 2% to about 90% of the level of the non-ionic surfactant can be substituted by a lipophilic surfactant, in another embodiment from about 25% to about 50%.

In one embodiment the emulsifier is Aerosol OT (sodium dioctyl sulfosuccinate) manufactured by Cytec.

### Whitening Index

In one embodiment, the present invention has a Whitening Index of from about 0.5 to about 4, in another embodiment from about 1 to about 4, in yet another embodiment from about 1.3 to about 3.5, and in another embodiment from about 1.5 to about 3. The Whitening Index is calculated as follows:
Db (for composition A) minus Db (for composition B) at treatment day 1, 2, 3 or 4 wherein composition A and composition B have the same concentration of bleaching agent, by weight of the composition, and composition A is a composition of the present invention (having the requisite amount of aqueous phase, emulsifier, hydrophobic phase, and bleach agent) and composition B does not.

Db for composition A is calculated as follows: 6-8 extracted human molars are cleaned and are mounted into Lego® blocks, and the front side of each molar is labeled to identify each molar. The molars are re-hydrated overnight in either water or phosphate buffer solution. Thereafter, the molars are removed from solution and then 0.03 to 0.1g of composition A is applied to the front surface of each molar. The molars are then placed in a 37 degree C incubator during the duration of the treatment. Each molar is treated with composition A for 30 minutes twice daily over a 4-day study period. After 30 minutes treatment time, the molars are removed from the incubator and are rinsed with distilled water to remove any residual composition. The molars are placed in the water or buffer solution in between each treatment. Two to four hours are allowed between each treatment period.

Digital images of the molars are obtained pretreatment (baseline) and after each total daily treatment (total daily treatment is 1 hour). Digital images of the molars are captured on computer using a high resolution digital camera (HC 1000 CCD®) manufactured by Fuji, Japan. Theses images are analyzed to derive numerical values for average tooth color in terms of standard CIELAB¹ three dimensional color space describing lightness/brightness from blue to yellow (b). Treatment efficacy after each day of treatment, b (calculated as the average b for each molar tested) is compared to the baseline b (calculated as the average b for each molar tested) for color measures b denoted as Db. In one embodiment the baseline b value of the extracted human molars ranges from about 10 to about 20, in another embodiment from about 12 to about 16.
¹ Commission Internationale de 1' Eclairage L*a*b* capable of representing all possible colors using three variables, a luninnce -L*; and color values on a red-green axis- a*; and a blue-yellow axis -b*.

The Whitening Index can be calculated at day 1 or 2 or 3 or 4, or it can be an average of the Db from days 1 to 4.

Db for composition B is calculated the same way as for composition A except that composition B is used instead of composition A.

### Optional Thickening Agents

The compositions herein optionally comprise a safe and effective amount of a thickening agent. In one embodiment the thickening agent (or viscosity modifier) functions to provide retention on the teeth or to increase retention of the composition on the teeth. The thickening agent may further provide acceptable rheology of the composition. The viscosity modifier may further function to inhibit settling and separation of components or control settling in a manner that facilitates re-dispersion and may control flow properties of the composition. A viscosity modifier is particularly useful to keep bleaching agents or other optional oral care active agents, that are in particulate form, suspended within the compositions of the present invention. The thickening agent herein may also serve as the adhesive means discussed herein below.

In one embodiment, when present, the thickening agent (viscosity modifier) is present at a level of from about 0.01% to about 20%, in one embodiment from about 0.1% to about 10%, in another embodiment from about 0.4% to about 5%, and in yet another embodiment from about 1% to about 3%, by weight of the composition.

Suitable viscosity modifiers herein include organo modified clays, silicas, synthetic polymers such as cellulose derivatives (e.g. methylcellulose, carboxymethylcellulose, hydroxyethylcellulose, hydroxypropylcellulose, hydroxypropylmethylcellulose, etc.), carbomer polymers (e.g. crosslinked polyacrylic acid copolymer or homopolymer and copolymers of acrylic acid cross linked with a polyalkenyl polyether), natural and synthetic gums, karaya gum, guar gum, gelatin, algin, sodium alginate, tragacanth, chitosan, polyethylene oxide, acrylamide polymers, polyacrylic acid, polyvinyl alcohol, polyamines, polyquarternary compounds, ethylene oxide polymers, polyvinylpyrrolidone, cationic polyacrylamide polymers, petrolatum, paraffin wax, microcrystalline waxes, polyethylene waxes, fumed silica, and mixtures thereof.

In one embodiment the thickening agent is selected from carbomers, e.g. the class of homopolymers of acrylic acid crosslinked with an alkyl ether of pentaerythritol or an alkyl ether of sucrose. Carbomers are commercially available from B.F. Goodrich as the Carbopol® series. In one embodiment the carbopols are Carbopol 934, 940, 941, 956, and mixtures thereof. Homopolymers of polyacrylic acid are described, for example, in U.S. Pat. No. 2,798,053. Other examples of homopolymers which are useful include Ultrez 10, ETD 2050, and 974P polymers, which are available from The B.F.Goodrich Company. Such polymers are homopolymers of unsaturated, polymerizable carboxylic monomers such as acrylic acid, methacrylic acid, maleic acid, itaconic acid, maleic anhydride, and the like.

### Viscosity of the Emulsion Composition

The composition of the present invention may be in the form of a viscous liquid or gels. In one embodiment the composition has a viscosity of from about 200 to about 1,000,000 cps at low shear rates (less than one 1/seconds). In another embodiment the viscosity is from about 100,000 to about 800,000 cps and in another embodiment from about 400,000 to about 600,000 cps. Viscosities are measured using standard Brookfield viscometer techniques at room temperature which are known to those skilled in the art. The viscosities of the final products were measured at 25 degrees C using a Brookfield viscometer (model DV-II) using Spindle D at 10 rpm. Three reading were recorded at 1 min intervals, the average being taken as the viscosity measure of the sample.

### Optional Colorants

Dyes, pigments, colorants, and mixtures thereof may optionally be included in the present invention to give the compositions herein colored appearance. An advantage of adding pigments and/or colorants to the compositions herein is that it will allow the user to see if the composition covers their teeth evenly and completely, since coverage is easier to see with a colored composition. In one embodiment the colorant provides color similar to the color of natural teeth. Colorants useful herein are stable with the bleach agent and are those recognized as safe.

The levels of dye, pigments and colorants that are optionally used herein are in the range of about 0.05% to about 20%, in another embodiment from about 0.10% to about 15% and in another embodiment from about 0.25% to about 5% by weight of the composition.

### Combination of Integral Carrier and Emulsion Composition

In one embodiment the present invention relates to a delivery system comprising the present compositions used with an integral carrier. In one embodiment the delivery system comprises: a first layer of a strip of material; a second layer comprising the present composition described herein, whereby the bleaching agent is releasably associated with the present composition and/or the strip of material. In one embodiment the present invention delivers whitening benefits to the oral cavity by directly applying the integral carrier to the teeth.

### 1. First Layer

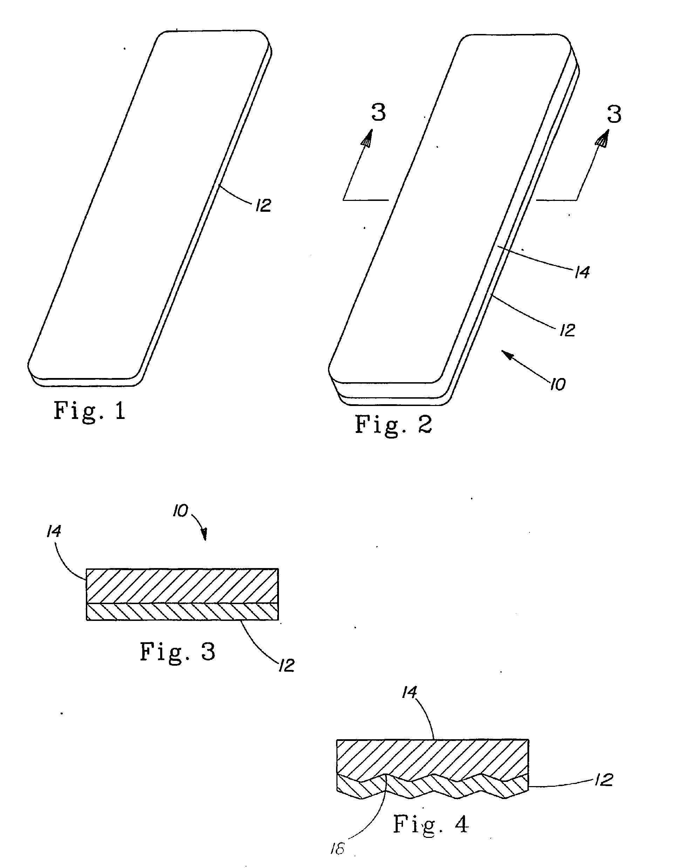

In one embodiment the first layer of the present invention comprises an integral carrier including a strip of material, dental tray, a sponge material, and mixtures thereof. Referring now to the drawings, and more particularly to FIGS. 1 and 2, there is shown a first embodiment of the present invention, generally indicated as 10, representing a delivery system for delivering bleach actives to the teeth and the oral cavity. Delivery system 10 has a strip of material 12, which is substantially flat, preferably with rounded corners. Releasably applied onto said strip of material 12 is a second layer composition 14. Second layer composition 14 is, in one embodiment, homogenous, and may be uniformly and continuously coated onto strip of material 12, as shown in FIG. 3. However, second layer composition 14 may alternatively be a continuous coating of second layer composition 14 along a longitudinal axis of a portion of strip of material 12. In addition second layer composition may be laminated or layered wherein the bleaching agent and/or adhesive means may not be present in all layers or laminates. In addition second layer composition may be an amorphous mixture of compositions wherein the bleaching agent and/or adhesive means may not be present in all phases of the amorphous mixture. In addition second layer composition may be applied as stripes, spots, and/or other patterns of the same or different compositions, wherein the bleaching agent and/or adhesive means may not be present in all stripes, spots, and/or other patterns of compositions.

As shown in FIG. 4 in an alternative embodiment, strip of material 12 may have shallow pockets 18 formed therein. When second layer composition 14 is coated on a strip of material 12, additional second layer composition 14, if present, fills shallow pockets 18 to provide reservoirs of second layer composition 14.

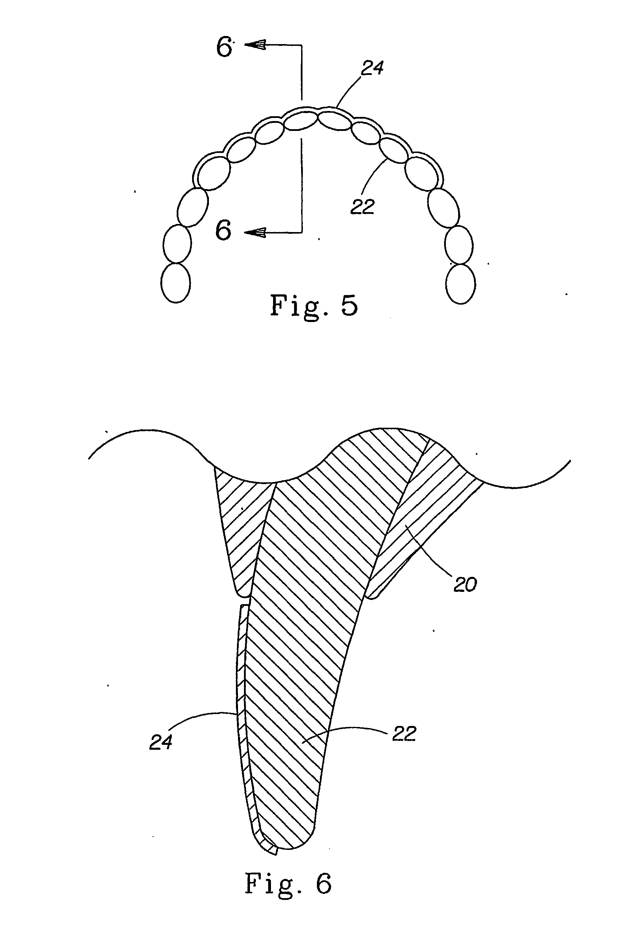

FIGS. 5 and 6 show a delivery system 24 of the present invention applied to the surface of a tooth and plurality of adjacent teeth. Embedded in adjacent soft tissue 20 are a plurality of adjacent teeth 22. Adjacent soft tissue herein defined as soft tissue surfaces surrounding the tooth structure including: papilla, marginal gingival, gingival sulculus, inter dental gingival, and gingival gum structure on lingual and buccal surfaces up to and including muco-gingival junction on the pallet.

In both FIGS. 5 and 6, delivery system 24 represents strip of material 12 and second layer composition 14, with second layer composition 14 on the side of strip material 12 facing tooth 22. Second layer composition 14 may be pre-applied to strip of material 12, or may be applied to strip of material 12 by the user prior to application to the teeth. In an alternate embodiment, the second layer composition may be applied directly to teeth 22 by the user and then covered by a strip of material 12. In any case, strip of material 12 has a thickness and flexural stiffness such that it can conform to the contoured surfaces of tooth 22 and to adjacent soft tissue 20. In one embodiment, the strip of material has sufficient flexibility to form to the contours of the oral surface, the surface being a plurality of adjacent teeth. The strip of material is also readily conformable to tooth surfaces and to the interstitial tooth spaces without permanent deformation when the delivery system is applied. The delivery system can be applied without significant pressure.

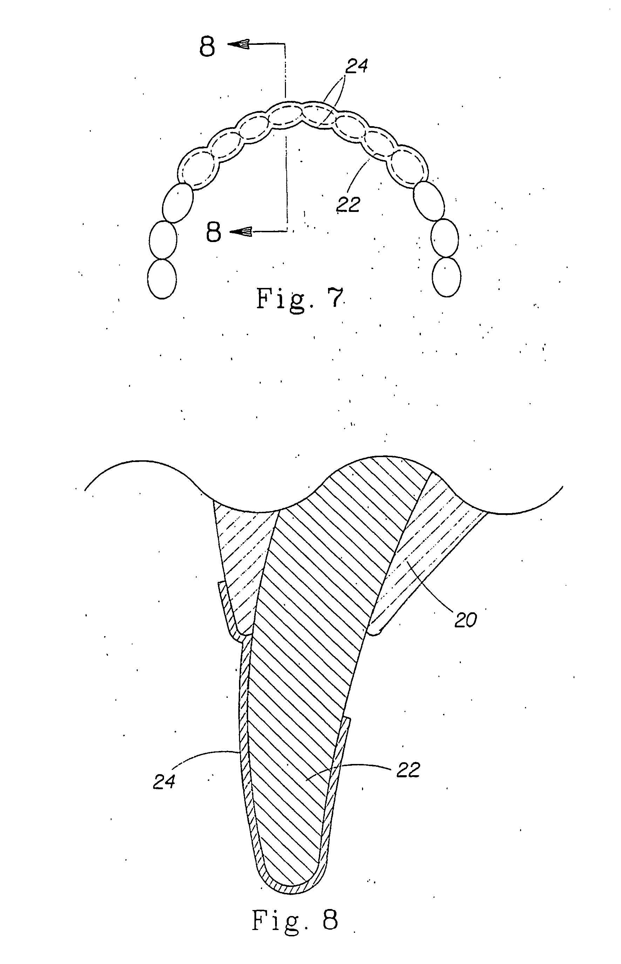

FIGS. 7 and 8 show a delivery system 24 of the present invention applied to both front and rear surfaces of a plurality of adjacent teeth 22 as well as to adjacent soft tissue 20. Delivery system 24 represents strip of material 12 and second layer composition 14, with second layer composition 14 on the side of strip of material 12 facing tooth 22.

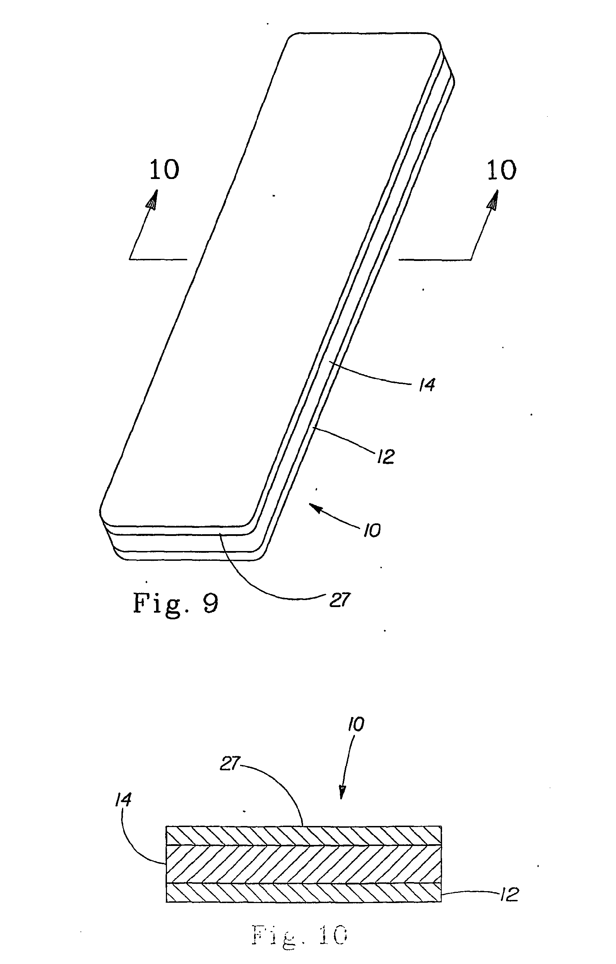

FIGS. 9 and 10 shows an optional release liner 27. Release liner 27 is attached to strip of material 12 by second layer composition 14. Second layer composition 14 is on the side of strip material 12 facing release liner 27. This side is applied to the tooth and gum surfaces once release liner 27 is removed.

In one embodiment the first layer of the delivery system of the present invention is comprised of a strip of material. Such first layer materials are described in more detail in U.S. Pat. Nos; 6,136,297; 6,096,328; 5,894,017; 5,891,453; and 5,879,691, all to Sagel, et al., and all assigned to The Procter & Gamble Company, and in U.S. Pat. Nos. 5,989,569 and 6,045,811 both to Dirksing, et al., and both assigned to The Procter & Gamble Company.

The strip serves as a protective barrier for the bleach. It prevents leaching and/or erosion of the second layer by for example, the wearer's tongue, lips, and saliva. This allows the active in the second layer to act upon the hard surfaces of the oral cavity for an extended period of time, from several minutes to several hours.

The strip material may comprise polymers, natural and synthetic woven materials, non-woven material, foil, paper, rubber and combinations thereof. The strip material may be a single layer of material or a laminate of more than one layer. Regardless of the number of layers, the strip of material is, in one embodiment, substantially water insoluble. The strip may also be water impermeable. In one embodiment the material is any type of polymer or combination of polymers that meet the required flexural rigidity and are compatible with oral care substances. Suitable polymers include, but are not limited to, polyethylene, ethylvinylacetate, polyesters, ethylvinyl alcohol and combinations thereof. Examples of polyesters include Molar® and fluoroplastics such as Teflon®, both manufactured by Dupont In one embodiment the material is polyethylene. The strip of material is generally less than 1 mm (millimeter) thick, in one embodiment less than 0.05 mm thick, in yet another embodiment from 0.001 to 0.03 mm thick. A polyethylene strip of material is generally less than 0.1 mm thick and in one embodiment is from 0.005 to 0.02 mm thick.

The shape of the strip of material is any shape and size that covers the desired oral surface. In one embodiment the strip has rounded corners to avoid irritation of the soft tissue of the oral cavity. "Rounded corners," means not having any sharp angles or points. In one embodiment, the length of the strip material is from about 2 cm (centimeter) to about 12 cm, in another embodiment from about 4 cm to about 9 cm. The width of the strip material will also depend on the oral surface area to be covered. The width of the strip is generally from about 0.5 cm to about 4 cm, in one embodiment from about 1 cm to about 2 cm. In yet another embodiment, the strip may be worn as a patch on one or several teeth to treat a localized condition.

The strip material may contain shallow pockets. When the composition is coated on a strip of material, bleach agents and/or oral care actives, fill shallow pockets to provide reservoirs of additional bleach agents and/or oral care actives. Additionally the shallow pockets help to provide texture to the delivery system. In one embodiment the strip of material will have an array of shallow pockets. Generally the shallow pockets are approximately 0.4 mm across and about 0.1 mm deep. When shallow pockets are included in the strip of material and compositions herein are applied to it in various thicknesses, the overall thickness of the delivery system is less than about 1 mm. In one embodiment the overall thickness is less than about 0.5 mm.

Flexural stiffness is a material property that is a function of a combination of strip thickness, width and material modulus of elasticity. This test is a method for measuring the rigidity of polyolefin film and sheeting. It determines the resistance to flexure of a sample by using a strain gauge affixed to the end of a horizontal beam. The opposite end of the beam presses across a strip of the sample to force a portion of the strip into a vertical groove in a horizontal platform upon which the sample rests. A microammeter wired to the strain gauge is calibrated in terms of deflection force. The rigidity of the sample is read directly from the microammeter and expressed as grams per centimeter of the sample strip width. In the present invention, the strip of material has a flexural stiffness of less than about 5 grams/cm as measured on a Handle-O-Meter, model #211-300, available from Thwing-Albert Instrument Company of Philadelphia, PA as per test method ASTM D2923-95. In one embodiment the strip has a flexural stiffness less than about 3 grams/cm, in another embodiment less than about 2 grams/cm and in yet another embodiment from about 0.1 to about 1 grams/cm. Generally, the flexural stiffness of the strip of material is substantially constant and does not change during normal use. For example, the strip of material does not need to be hydrated for the strip to achieve the low flexural stiffness in the above-specified ranges.

This relatively low stiffness enables the strip of material to cover the contours of the oral surface with very little force being exerted. That is, conformity to the contours of the oral surface of the wearer's mouth is maintained because there is little residual force within the strip of material to cause it to return to its shape just prior to its application to the oral surface, i.e. substantially flat. The strip of material's flexibility enables it to contact soft tissue over an extended period of time without irritation. The strip of material does not require continuous pressure for retention against the oral surface.

In one embodiment the delivery systems herein comprise an adhesion means and are capable of adhesion to oral surfaces especially the teeth. This adhesion means may be provided by the present compositions herein or the adhesion means is provided independently of the compositions herein (for example the adhesion means is a separate phase from the compositons herein where the compositions may or may not also have an adhesive means). In once embodiment the strip of material is held in place on the oral surface by adhesive attachment provided by the present composition. The viscosity and general tackiness of the aqueous emulsion to dry surfaces cause the strip to be adhesively attached to the oral surface without substantial slippage from the frictional forces created by the lips, teeth, tongue, and other oral surfaces rubbing against the strip of material while talking drinking, etc. However, this adhesion to the oral surface is low enough to allow the strip of material to be easily removed by the wearer by simply peeling off the strip of material using one's finger. The delivery system is easily removable from the oral surfaces without the use of an instrument, a chemical solvent or agent or excess friction.

In another embodiment the strip of material is held in place on the oral surface by adhesive means and attachment provided by the integral carrier itself. In one embodiment the strip of material can extend, attach, and adhere to the oral soft tissue. Alternatively, an adhesive can be applied to that portion of the strip of material that will attach the delivery systems to the oral soft tissue. In another embodiment the strip of material is held in place by an adhesion means that is independent of the composition of the present inventions herein, as disclosed in WO 03/015656, published Feb. 27,2003, SmithKline Beecham.

Examples of adhesion means being provided independent of the compositions herein include the following.

In one embodiment, the composition and an adhesive material may be deposited in separate discrete locations in relation to the strip surface. In one embodiment the composition and adhesive may be deposited on the surface of the strip in respective spatially separated places on the surface. For example the adhesive may be deposited in places on the strip surface that enable part of the strip to stick to an oral surface adjacent to a tooth surface, e.g. a gum surface, so that another part of the strip on which the composition is deposited or into which it is impregnated may contact the tooth surface.

Alternatively the adhesive and composition may be spatially separated but both in locations that enable the adhesive and composition to contact the same type of tissue, e.g. tooth or gum surface, respective discrete spots or patches on the surface, that are relatively small. For example parallel lines of the adhesive and composition, one or more patches of composition bordered partly or completely surrounded by a border of the adhesive, a single large patch covering substantially the entire surface of the strip and bordered partly or completely by a line of the adhesive. Adhesive may be deposited on one or more patch bordered partly or completely surrounded by a border of the composition.

In another embodiment the composition and/or adhesive may be encapsulated. Encapsulation may for example be in micro-capsules, or macro-capsules. Methods of micro-encapsulation are known, for example in which a droplet of a substance in a liquid phase is enclosed within a layer of an encapsulation material, and then separated from the liquid. Such capsules may be deposited on or adjacent the surface of the integral carrier, and may for example be burst physically or chemically, e.g. by pressure e.g. as the strip is applied to the tooth surface or by subsequent bit action, by breaching of the capsule wall under the action of the temperature, moisture, pH, chemicals or enzymes in the mouth environment etc. For example respective capsules of composition and adhesive may be attached to the surface of the strip, e.g. by means of a second adhesive or by embedding the capsules in the strip of material. For example a thin layer of the adhesive may be deposited on the surface of the strip, and capsules of the composition may be embedded at least partly if not completely within this adhesive layer, or may sit upon the surface of this adhesive layer.

In another embodiment the adhesive may be provided in granules, e.g. pellets or micropellets, which may release their content under the influence of the mouth environment, for example moisture, chemicals or enzymes in the mouth, and may be coated to achieve this release. Methods of granulation and palletizing are known, as are coating polymers such as the know Eudragit™ polymers which dissolve at specified pH. Such adhesive granules may be deposited on or adjacent the surface of the strip. For example capsules and/or granules of adhesive may be located substantially uniformly over the strip surface, or alternatively respective capsules and/or granules of adhesive may be situated at separate respective locations on the surface of the strip.

In another embodiment a layer of the composition may be deposited relatively proximal to, e.g. adjacent to and in contact with the surface, and a layer of the adhesive may be deposited relatively distal from the surface e.g. adjacent to and in contact with the underlying layer of composition. In such a construction the adhesive may stick the strip to the tooth surface, and the composition may pass through the adhesive layer, for example as the adhesive layer becomes permeable under the influence of the mouth environment. The adhesive layer may in such a construction have one or more holes passing through the layer to facilitate the passage of the composition through the adhesive layer. Alternatively for example a layer of the adhesive may be deposited relatively proximal to, e.g. adjacent to and in contact with the surface, and a layer of the composition may be deposited relatively distal from the surface e.g. adjacent to and in contact with an underlying layer of adhesive. In such a construction the layer of composition may need one or more holes passing through the layer to facilitate the passage of the adhesive through the composition. In the above constructions the passage of material from the underlying layer may be facilitated by pressure as the strip is applied to the tooth surface.

Mechanical adhesive means may also be used to provide an adhesive function, used either alone or in combination with any other adhesive device disclosed herein. In another embodiment mechanical adhesion between the strip and tooth or other oral surface is provided by the strip comprising a plastically deformable material, which can be plastically deformed by the user to conform the strip to the contours of the tooth or other oral surface, and so adhere thereto by mechanical gripping. Such gripping may be enhanced by e.g. surface effects between the strip and the surface such as formation of a partial vacuum or surface tension effects. For example the strip may have anchors on its surface, positioned at approximately the spacings of gaps between teeth, and these anchors may fit into the gaps between the teeth. For example the surface of the strip which is to contact the tooth surface may be provided with micro-suckers, that is a plurality of small cavities in the surface of the strip which can be pressed onto the tooth surface to drive air out therefrom, and thereby create a partial vacuum, so that the strip is thereafter held on the tooth surface by air pressure. Such anchors or micro-suckers may be located on the surface of a strip which is to contact the tooth surface. Such a strip may for example be stretchable, so that it can be adjusted to the spacings of gaps between an individual user's teeth. Another form of "mechanical" adhesion may be provided by a strip which shrinks in contact with the tooth surface, so it can physically grip the surface of the tooth.

When the adhesive means is provided by an adhesive, the adhesive may be any adhesive which may be used to stick materials to the tooth surface or to a surface of the oral cavity surfaces. Suitable adhesives include skin, gum and muco adhesives, and should be able to withstand the moisture, chemicals and enzymes of the oral environment for long enough for the oral care actives and/or bleach to take effect, but may be soluble and/or biodegradable thereafter. Suitable adhesives may for example comprise water soluble polymers, hydrophobic and/or non-water soluble polymers, pressure and moisture sensitive adhesives, e.g. dry adhesives which become tacky upon contact with the mouth environment, e.g. under the influence of moisture, chemicals or enzymes etc. in the mouth. Suitable adhesives include natural gums, synthetic resins, natural or synthetic rubbers, those gums and polymers listed above under "Thickening Agents", and various other tacky substances of the kind used in known adhesive tapes, those known from US-A-2,835,628.

### Second Layer

In one embodiment the second layer comprises a safe and effective amount of the present composition described herein.

### Optional Release Liner

The release liner may be formed from any material which exhibits less affinity for the second layer composition than the second layer composition exhibits for itself and for the first layer strip of material. The release liner may comprise a rigid sheet of material such as polyethylene, paper, polyester, or other material, which is then coated with a nonstick type material. The release liner may be cut to substantially the same size and shape as the strip of material or the release liner may be cut larger than the strip of material to provide a readily accessible means for separating the material from the strip. The release liner may be formed from a brittle material that cracks when the strip is flexed or from multiple pieces of material or a scored piece of material. Alternatively, the release liner may be in two overlapping pieces such as a typical adhesive bandage design. A description of materials suitable as release agents is found in Kirk-Othmer, Encyclopedia of Chemical Technology, Fourth Edition, Volume 21, pp. 207-218.

### Combination of Soft or Rigid Dental Trays or Foam Materials and Emulsion Composition

The present compositions may be used in combination with an integral carrier including a dental tray and/or foam material. Dental trays are well known in the whitening art. The general process for preparing dental trays is known in the art. For example, an alginate impression which registers all teeth surfaces plus gingival margin is made and a stone cast is promptly made of the impression. If reservoirs are desired they are prepared by building a layer of rigid material on the stone cast on specific teeth surfaces to be treated. A dental tray is then vacuum formed from the modified cast using conventional techniques. Once formed, the tray is preferably trimmed barely shy of the gingival margin on both buccal and lingual surfaces. Enough tray material should be left to assure that all of the tooth will be covered to within about ¼ to about 1/3 mm of the gingival border upon finishing and beveling the tray periphery. In one embodiment one can scallop up and around interdental papilla so that the finished tray does not cover them. All tray edges are preferably smoothed so that the lip and tongue will not feel an edge prominence. The resulting tray, in one embodiment, provides a perfect fit of the patient's teeth optionally with reservoirs or spaces located where the rigid material was placed on the stone cast. Dental trays may comprise of soft transparent vinyl material having a preformed thickness from 1mm (0.04 inch) to 1.5mm (0.06 inch). Soft material is more comfortable for the patient to wear. Harder material (or thicker plastic) may also be used to construct the tray.

Dentists have traditionally utilized three types of dental appliances for bleaching teeth. The first type is a rigid appliance which is fitted precisely to the patient's dental arches. A second type of rigid custom dental appliance is an "oversized" rigid custom dental appliance. The fabrication of rigid, custom dental appliances entails fabricating stone models of the patient's dental arch impressions, and heating and vacuum-forming a thermoplastic sheet to correspond to the stone models of a patient's dental arches. Thermoplastic films are sold in rigid or semi rigid sheets, and are available in various sizes and thickness. The dental laboratory fabrication technique for the oversized rigid dental appliance involves augmenting the facial surfaces of the teeth on the stone models with materials such as die spacer or light cured acrylics. Next, thermoplastic sheeting is heated and subsequently vacuum formed around the augmented stone models of the dental arch. The net effect of this method results in an "oversized" rigid custom dental appliance.

A third type of rigid custom dental appliance, used with less frequency, is a rigid bilaminated custom dental appliance fabricated from laminations of materials, ranging from soft porous foams to rigid, non-porous films. The non-porous, rigid thermoplastic shells of these bilaminated dental appliances encase and support an internal layer of soft porous foam.

A fourth type of dental tray replaces rigid custom dental appliances with disposable U-shaped soft foam trays, which may be individually packaged, and which may be saturated with a pre-measured quantity of the composition of the present invention. The soft foam material is generally an open celled plastic material. Such a device is commercially available from Cadco Dental Products in Oxnard, Calif. under the tradename VitalWhite™. In one embodiment these soft foam trays comprise a backing material (e.g. a closed cell plastic backing material) to minimize the elution of the bleaching agent from the device, into the oral cavity to minimize ingestion by the patient and/or irritation of the oral cavity tissues. In another embodiment the soft foam tray is encased by a nonporous flexible polymer. In another embodiment the open cell foam is attached to the frontal inner wall of the dental appliance and/or the open cell foam is attached to the rear inner wall of the dental appliance.

Those of ordinary skill in the art will readily recognize and appreciate, that the present compositions must be thick enough not to simply run out between the open cell structure of the foam and must be thin enough to slowly pass through the open cell foam over time. In other words, the open cell foam material has an internal structural spacing sized relative to the viscosity of the compositions to absorb and allow the composition to pass therethrough.

An example of a closed cell material is a closed-celled polyolefin foam sold by the Voltek division of Sekisui America Corporation of Lawrence, Mass. under the tradename Volora which is from 0.75mm (1/32") to 3mm(1/8") in thickness. A closed cell material may also comprise of a flexible polymeric material.

An example of an opened cell material is an open celled polyethylene foam sold by the Sentinel Foam Products division of Packaging Industries Group, Inc. of Hyannis, Mass. under the tradename Opcell which is from 1.5mm (1/16") to 9mm (3/8") in thickness. Other open cell foam useful herein include hydrophilic open foam materials such as hydrogel polymers (e.g Medicell™ foam available from Hydromer, Inc. Branchburg, J.J.). Open cell foam may also be hydrophilic open foam material imbibed with agents to impart high absorption of fluids, such as polyurethane or polyvinylpyrrolidone chemically imbibed with various agents.

Appliances of the above type are further described in US Patent Nos. 5,980,249, M.G. Fontenot, and US 5,575,654, M.G. Fontenot.

The above dental appliances may be designed to be disposable or reuseable. Further dental trays are disclosed in U.S. Patent 6,368,576, Steven D. Jensen, issued April 9, 2002; U.S. Patent 6,309,625 Jensen, et al., issued Oct 30, 2001; U.S. Patent 6,183,251, Dan E. Fischer, issued February 6, 2001; U.S. Patent 6,036,943, Dan E. Fischer, issued March 14,2000; U.S. Patent 5,985,249, Dan E. Fischer, issued November 16, 1999; U.S. Patent 5,846,058, Dan E. Fischer, issued Dec 8, 1998; U.S. Patent 6,382,979, Sherrill F. Lindquist, issued May 7, 2002; U.S. Patent 5,098,303, Fischer, issued March 24, 1992, and U.S. Patent 5,855,870, Dan E. Fischer, issued January 5, 1999.

### Optional Oral Care Active Agents

The present invention may optionally comprise a safe and effective amount of an oral care active agent selected from the group consisting of anticalculus agent, fluoride ion source, antimicrobial agents, dentinal desensitizing agents, anesthetic agents, antifungal agents, anti-inflammatory agents, selective H-2 antagonists, anticaries agents, nutrients, and mixtures thereof. The oral care active agent preferably contains an active at a level where upon directed use, the benefit sought by the wearer is promoted without detriment to the oral surface to which it is applied. Examples of the oral conditions these actives address include, but, are not limited to, appearance and structural changes to teeth, whitening, stain bleaching, stain removal, plaque removal, tartar removal, cavity prevention and treatment, inflamed and/or bleeding gums, mucosal wounds, lesions, ulcers, aphthous ulcers, cold sores, tooth abscesses, and the elimination of mouth malodor resulting from the conditions above and other causes such as microbial proliferation.

Suitable oral care actives include any material that is generally considered safe for use in the oral cavity and that provides changes to the overall appearance and/or health of the oral cavity. The level of oral care substance in the compositions of the present invention is generally, unless specifically noted, from about 0.01% to about 50%, in one embodiment from about 0.1% to about 20%, in another embodiment from about 0.5% to about 10%, and in another embodiment from about 1% to about 7%, by weight of the composition.

Oral care compositions or substances of the present invention may include many of the actives previously disclosed in the art. The following is a non-limiting list of oral care actives that may be used in the present invention.

### Anticaries Agents and Fluoride Ion Source

The present composition may comprise a safe and effective amount of an anticaries agent, and mixtures thereof. In one embodiment the anticaries agent is selected from the group consisting of xylitol, fluoride ion source, and mixtures thereof. The fluoride ion source provides free fluoride ions during the use of the composition. In one embodiment the oral care active agent is a fluoride ion source selected from the group consisting of sodium fluoride, stannous fluoride, indium fluoride, organic fluorides such as amine fluorides, and sodium monofluorophosphate. Sodium fluoride is the fluoride ion in another embodiment. Norris et al., U.S. Patent 2,946,725, issued July 26, 1960, and Widder et al., U.S. Patent 3,678,154 issued July 18, 1972, disclose such fluoride salts as well as others that can be used as the fluoride ion source.

Preferably the instant compositions provide from about 50 ppm to 10,000 ppm, more preferably from about 100 to 3000 ppm, of fluoride ions in the compositions that contact dental surfaces when used with the delivery system of the present invention Anticalculus Agents

The present compositions may comprise a safe and effective amount of at least one anticalculus agent. This amount is generally from about 0.01% to about 40% by weight of the composition, in another embodiment is from about 0.1% to about 25%, and in yet another embodiment is from about 4.5% to about 20%, and in yet another embodiment is from about 5% to about 15%, by weight of the composition. The anticalculus agent should also be essentially compatible with the other components of the composition.

The anticalculus agent is selected from the group consisting of polyphosphates and salts thereof; polyamino propane sulfonic acid (AMPS) and salts thereof; polyolefin sulfonates and salts thereof; polyvinyl phosphates and salts thereof; polyolefin phosphates and salts thereof; diphosphonates and salts thereof; phosphonoalkane carboxylic acid and salts thereof; polyphosphonates and salts thereof; polyvinyl phosphonates and salts thereof; polyolefin phosphonates and salts thereof; polypeptides; and mixtures thereof. In one embodiment, the salts are alkali metal salts. In another embodiment the anticalculus agent is selected from the group consisting of polyphosphates and salts thereof; diphosphonates and salts thereof; and mixtures thereof. In another embodiment the anticalculus agent is selected from the group consisting of pyrophosphate, polyphosphate, and mixtures thereof.

### Polyphosphate

In one embodiment of the present invention, the anticalculus agent is a polyphosphate. A polyphosphate is generally understood to consist of two or more phosphate molecules arranged primarily in a linear configuration, although some cyclic derivatives may be present. Linear polyphosphates correspond to (X PO₃)ₙ where n is about 2 to about 125, wherein preferably n is greater than 4, and X is for example sodium, potassium, etc. For (X PO₃)ₙ when n is at least 3 the polyphosphates are glassy in character. Counterions for these phosphates may be the alkali metal, alkaline earth metal, ammonium, C₂-C₆ alkanolammonium and salt mixtures. Polyphosphates are generally employed as their wholly or partially neutralized water soluble alkali metal salts such as potassium, sodium, ammonium salts, and mixtures thereof. The inorganic polyphosphate salts include alkali metal (e.g. sodium) tripolyphosphate, tetrapolyphosphate, dialkyl metal (e.g. disodium) diacid, trialkyl metal (e.g. trisodium) monoacid, potassium hydrogen phosphate, sodium hydrogen phosphate, and alkali metal (e.g. sodium) hexametaphosphate, and mixtures thereof. Polyphosphates larger than tetrapolyphosphate usually occur as amorphous glassy materials. In one embodiment the polyphosphates are those manufactured by FMC Corporation which are commercially known as Sodaphos (n ≈6), Hexaphos (n≈13), and Glass H (n≈21), and mixtures thereof. The present compositions will typically comprise from about 0.5% to about 20%, in one embodiment from about 4% to about 15%, in yet another embodiment from about 6% to about 12%, by weight of the composition of polyphosphate.

The phosphate sources are described in more detail in Kirk & Othmer, Encyclopedia of Chemical Technology, Fourth Edition, Volume 18, Wiley-Interscience Publishers (1996), pages 685-707, incorporated herein by reference in its entirety, including all references incorporated into Kirk & Othmer.

In one embodiment the polyphosphates are the linear "glassy" polyposphates having the formula:

XO(XPO₃)ₙX

wherein X is sodium or potassium; and n averages from about 6 to about 125.

In one embodiment, when n is at least 2 in either of the above polyphosphate formulas, the level of anticalculus agent is from about 4.5% to about 40%, in another embodiment is from about 5% to about 25%, and in even another embodiment is from about 8% to about 15%, by weight of the composition. Polyphosphates are disclosed in US 4,913,895, herein incorporated by reference.

### Pyrophosphate

The pyrophosphate salts useful in the present compositions include, alkali metal pyrophosphates, di-, tri-, and mono-potassium or sodium pyrophosphates, dialkali metal pyrophosphate salts, tetraalkali metal pyrophosphate salts, and mixtures thereof. In one embodiment the pyrophosphate salt is selected from the group consisting of trisodium pyrophosphate, disodium dihydrogen pyrophosphate (Na₂H₂P₂O₇), dipotassium pyrophosphate, tetrasodium pyrophosphate (Na₄P₂O₇), tetrapotassium pyrophosphate (K₄P₂O₇), and mixtures thereof. The pyrophosphate salts described in U.S. Patent 4,515,772, issued May 7, 1985, and US Pat. No. 4,885,155, issued December 5, 1989, both to Parran et al., are incorporated herein by reference in their entirety, as well as the references disclosed therein. The pyrophosphate salts are described in more detail in Kirk & Othmer, Encyclopedia of Chemical Technology, Third Edition, Volume 17, Wiley-Interscience Publishers (1982), pages 685-707.

In one embodiment, the compositions of the present invention comprise tetrasodium pyrophosphate. Tetrasodium pyrophosphate may be the anhydrous salt form or the decahydrate form, or any other species stable in solid form in the present compositions. The salt is in its solid particle form, which may be its crystalline and/or amorphous state, with the particle size of the salt preferably being small enough to be aesthetically acceptable and readily soluble during use.

The level of pyrophosphate salt in the compositions of the present invention is any safe and effective amount, and is generally from about 1.5% to about 15%, in another embodiment from about 2% to about 10%, and yet in another embodiment from about 3 % to about 8%, by weight of the composition.

### Other Anticalculus Agents

Polyolefin sulfonates include those wherein the olefin group contains 2 or more carbon atoms, and salts thereof. Polyolefin phosphonates include those wherein the olefin group contains 2 or more carbon atoms. Polyvinylphosphonates include polyvinylphosphonic acid. Diphosphonates and salts thereof include azocycloalkane-2,2-diphosphonic acids and salts thereof, ions of azocycloalkane-2,2-diphosphonic acids and salts thereof (such as those which the alkane moiety has five, six or seven carbon atoms, in which the nitrogen atom is unsubstituted or carries a lower alkyl substitutent, e.g. methyl), azacyclohexane-2,2-diphosphonic acid, azacyclopentane-2,2-diphosphonic acid, N-methyl-azacyclopentane-2,3-diphosphonic acid, EHDP (ethanehydroxy-1,1,-diphosphonic acid), AHP (azacycloheptane-2,2-diphosphonic acid, a.k.a. 1-azocycloheptylidene-2,2-diphosphonic acid), ethane-1 -amino-1,1 -diphosphonate, dichloromethane-diphosphonate, etc. Phosphonoalkane carboxylic acid or their alkali metal salts include PPTA (phosphonopropane tricarboxylic acid), PBTA (phosphonobutane-1,2,4-tricarboxylic acid), each as acid or alkali metal salts. Polyolefin phosphates include those wherein the olefin group contains 2 or more carbon atoms. Polypeptides include polyaspartic and polyglutamic acids.

Azacycloalkane-2,2-diphosphonic acids are disclosed in US 3,941,772, issued March 2, 1976, Ploger et al., assigned to Henkel and US 3,988,443, issued Oct. 26, 1976, Ploger et al.

Optional agents to be used in place of or in combination with the pyrophosphate salt include such known materials as synthetic anionic polymers, including polyacrylates and copolymers of maleic anhydride or acid and methyl vinyl ether (e.g., Gantrez), as described, for example, in U.S. Patent 4,627,977, to Gaffar et al.; as well as, e.g., polyamino propoane sulfonic acid (AMPS), zinc citrate trihydrate, polyphosphates (e.g., tripolyphosphate; hexametaphosphate), diphosphonates (e.g., EHDP; AHP), polypeptides (such as polyaspartic and polyglutamic acids), and mixtures thereof.

### Antimicrobial Agents

Antimicrobial antiplaque agents may also by optionally present in the present compositions. Such agents may include, but are not limited to, triclosan, 5-chloro-2-(2,4-dichlorophenoxy)-phenol, as described in The Merck Index, 11th ed. (1989), pp. 1529 (entry no. 9573) in U.S. Patent No. 3,506,720, and in European Patent Application No. 0,251,591 of Beecham Group, PLC, published January 7, 1988; chlorhexidine (Merck Index, no. 2090), alexidine (Merck Index, no. 222; hexetidine (Merck Index, no. 4624); sanguinarine (mercy Index, no. 8320); benzalkonium chloride (Merck Index, no. 1066); salicylanilide (Merck Index, no. 8299); domiphen bromide (Merck Index, no. 3411); cetylpyridinium chloride (CPC) (Merck Index, no. 2024; tetradecylpyridinium chloride (TPC); N-tetradecyl-4-ethylpyridinium chloride (TDEPC); octenidine; delmopinol, octapinol, and other piperidino derivatives; effective antimicrobial amounts of essential oils and combinations thereof for example citral, geranial, and combinations of menthol, eucalyptol, thymol and methyl salicylate; antimicrobial metals and salts thereof for example those providing zinc ions, stannous ions, copper ions, and/or mixtures thereof; bisbiguanides, or phenolics; antibiotics such as augmentin, amoxicillin, tetracycline, doxycycline, minocycline, and metronidazole; and analogs and salts of the above antimicrobial antiplaque agents; anti-fungals such as those for the treatment of *candida albicans*. If present, these agents generally are present in a safe and effective amount for example from about 0.1% to about 5% by weight of the compositions of the present invention.

### Antiinflammatory Agents

Anti-inflammatory agents may also be present in the oral compositions of the present invention. Such agents may include, but are not limited to, non-steroidal anti-inflammatory agents such as aspirin, ketorolac, flurbiprofen, ibuprofen, naproxen, indomethacin, aspirin, ketoprofen, piroxicam and meclofenamic acid, COX-2 inhibitors such as valdecoxib, celecoxib and rofecoxib, and mixtures thereof. If present, the anti-inflammatory agents generally comprise from about 0.001% to about 5% by weight of the compositions of the present invention. Ketorolac is described in U.S. Patent 5,626,838, issued May 6, 1997.

### H-2 Antagonists

The present invention may also include a safe and effective amount of a selective H-2 antagonist. Selective H-2 antagonists include compounds which are disclosed in U.S. Patents 5,294,433 and 5,364,616 Singer et al., issued 3/15/94 and 11/15/94 respectively and assigned to Procter & Gamble, wherein the selective H-2 antagonist is selected from the group consisting of cimetidine, etintidine, ranitidine, ICIA-5165, tiotidine, ORF-17578, lupitidine, donetidine, famotidine, roxatidine, pifatidine, lamtidine, BL-6548, BMY-25271, zaltidine, nizatidine, mifentidine, BMY-25368 (SKF-94482), BL-6341A, ICI-162846, ramixotidine, Wy-45727, SR-58042, BMY-25405, loxtidine, DA-4634, bisfentidine, sufotidine, ebrotidine, HE-30-256, D-16637, FRG-8813, FRG-8701, impromidine, L-643728, and HB-408. Particularly preferred is cimetidine (SKF-92334), N-cyano-N'-methyl-N"-(2-(((5-methyl-1H-imidazol-4-yl)methyl)thio)ethyl)guanidine:

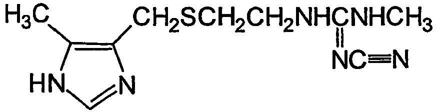

Cimetidine is also disclosed in the Merck Index, 11th edition (1989), p. 354 (entry no. 2279), and Physicians' Desk Reference, 46th edition (1992), p. 2228. Related preferred H-2 antagonists include burimamide and metiamide.

### Nutrients

Nutrients may improve the condition of the oral cavity and can be included in the compositions of the present invention. Nutrients include minerals, vitamins, oral nutritional supplements, enteral nutritional supplements, and mixtures thereof.

Minerals that can be included with the compositions of the present invention include calcium, phosphorus, fluoride, zinc, manganese, potassium and mixtures thereof. These minerals are disclosed in Drug Facts and Comparisons (loose leaf drug information service), Wolters Kluer Company, St. Louis, Mo., ©1997, pp10-17.

Vitamins can be included with minerals or used separately. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Such vitamins are disclosed in Drug Facts and Comparisons (loose leaf drug information service), Wolters Kluer Company, St. Louis, Mo., ©1997, pp. 3-10.

Oral nutritional supplements include amino acids, lipotropics, fish oil, and mixtures thereof, as disclosed in Drug Facts and Comparisons (loose leaf drug information service), Welters Kluer Company, St. Louis, Mo., ©1997, pp. 54-54e. Amino acids include, but, are not limited to L-Tryptophan, L-Lysine, Methionine, Threonine, Levocarnitine or L- carnitine and mixtures thereof. Lipotropics include, but, are not limited to choline, inositol, betaine, linoleic acid, linolenic acid, and mixtures thereof. Fish oil contains large amounts of Omega-3 (N-3) Polyunsaturated fatty acids, eicosapentaenoic acid and docosahexaenoic acid.

Antioxidants that may be included in the oral care composition or substance of the present invention include, but are not limited to Vitamin E, ascorbic acid, Uric acid, carotenoids, Vitamin A, flavonoids and polyphenols, herbal antioxidants, melatonin, aminoindoles, lipoic acids and mixtures thereof.

Enteral nutritional supplements include, but, are not limited to protein products, glucose polymers, corn oil, safflower oil, medium chain triglycerides as disclosed in Drug Facts and Comparisons (loose leaf drug information service), Wolters Kluer Company, St. Louis, Mo., ©1997, pp. 55-57.

### Desensitizing Agents

Anti-pain or desensitizing agents can also be present in the oral care compositions of the present invention. Such agents may include, but are not limited to, strontium chloride, potassium nitrate, natural herbs such as gall nut, Asarum, Cubebin, Galanga, scutellaria, Liangmianzhen, Baizhi, etc.

### Optional Flavoring Agents

The compositions of the present invention also optionally comprise a safe and effective amount of a flavoring agent. Suitable flavoring agents include oil of wintergreen, oil of peppermint, oil of spearmint, clove bud oil, menthol, anethole, methyl salicylate, eucalyptol, 1-menthyl acetate, sage, eugenol, parsley oil, oxanone, alphairisone, marjoram, lemon, orange, propenyl guaethol, cinnamon, vanillin, thymol, linalool, cinnamaldehyde glycerol acetal known as CGA, and mixtures thereof. If present the flavoring agents are generally used in the present compositions at levels of from about 0.01% to about 30%, in another embodiment from about 1% to about 20%, in yet another embodiment from about 1.5% to about 15%, by weight of the composition.

The present compositions may optionally comprise sweetening agents including sucralose, sucrose, glucose, saccharin, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate and sodium saccharin, and mixtures thereof. If present, the composition contains from about 0.1% to about 10% of these agents, in another embodiment from about 0.1% to about 1%, by weight of the composition.

Coolants, salivating agents, warming agents, and numbing agents can be used as optional ingredients in compositions of the present invention, in one embodiment at a level of from about 0.001% to about 10%, in another embodiment from about 0.1% to about 1%, by weight of the composition.

The coolant can be any of a wide variety of materials. Included among such materials are carboxamides, menthol, ketals, diols, and mixtures thereof. In one embodiment optional coolants in the present compositions are the paramenthan carboxyamide agents such as N-ethyl-p-menthan-3-carboxamide, known commercially as "WS-3", N,2,3-trimethyl-2-isopropylbutanamide, known as "WS-23," and mixtures thereof. Coolants may also be selected from the group consisting of menthol, 3-1-menthoxypropane-1,2-diol known as TK-10 manufactured by Takasago, menthone glycerol acetal known as MGA manufactured by Haarmann and Reimer, and menthyl lactate known as Frescolat® manufactured by Haarmann and Reimer. The terms menthol and menthyl as used herein include dextro- and levorotatory isomers of these compounds and racemic mixtures thereof. TK-10 is described in U.S. Pat. No. 4,459,425, Amano et al., issued 7/10/84. WS-3 and other agents are described in U.S. Pat. No. 4,136,163, Watson, et al., issued Jan. 23, 1979.

In one embodiment salivating agents include Jambu® manufactured by Takasago. Warming agents include capsicum and nicotinate esters, such as benzyl nicotinate. Numbing agents include benzocaine, lidocaine, clove bud oil, and ethanol.

### Methods of Manufacturing Emulsion Composition or Delivery Systems

Preparation of emulsions is well known in the art. See for example, Remmingtion: the Science and Practice of Pharmacy, 19th ed., Vol. II, Chapters 20, 80, 86, etc. Generally, the components are separated into those that are oil-soluble and those that are water-soluble. These are dissolved in their respective solvents by heating. The two phases are then mixed and the product is stirred and cooled. After combining the phases, the present emulsions may be agitated or sheared by methods, including shaking, intermittent shaking, high shear mixing, or by using high speed mixers, blenders, colloid mills, homogenizers, or ultrasonic techniques. Various test methods are available to confirm the type of emulsion prepared. These test methods include the dilution test, conductivity test, and the dye-solubility test methods. Since some test methods may give incorrect results, confirmation of the type of emulsion by one test should be confirmed by another method as well. Further description of test methods are disclosed in Remington: The Science and Practice of Pharmacy, 19th ed., volume 1, 1995, pp. 282-283.

In the above described delivery system, the second layer composition is suitably made as follows: Combine the aqueous phase, the inert hydrophobic phase and the bleach active into a mixing vessel and mix well with any means known within the art, for example, with spatula or mixer. Heat the composition, if desired, to facilitate mixing. Continue mixing the composition until homogenous. Where the active is included in solid particulate form, the addition of an optional viscosity modifier, such as silica, may be appropriate to keep the particulate dispersed and suspended within the composition. Flavorants or sweeteners may also be added to the second layer composition by mixing as desired. Thereafter the composition is added to the integral carrier, as desired.

The integral carrier, such as a strip, may be formed by several of the film making processes known in the art. In one embodiment a strip of polyethylene is made by a blown process or a cast process. Other processes including extrusion or processes that do not affect the flexural rigidity of the strip of material are also feasible. Additionally, the second layer composition may be incorporated onto the strip during the processing of the strip. The second layer composition may be a laminate on the strip.

### Methods of Using the Compositions and/or Delivery Systems

The present invention can be applied to the teeth of a consumer in the dental office by a dental professional, or can be used at home by the consumer. Generally, the recommended treatment period is, in one embodiment, a sufficient period of time to achieve whitening.

In practicing the present invention, the user applies the composition herein that contains the bleach to obtain the desired effect, e.g., whitening, to one or more teeth. The composition can be applied with a paint-on device, a syringe or unit dose syringe, squeezable tube, a brush, a pen or brush tip applicator, a doe's foot applicator, or the like, or even with the fingers. The composition can also be combined with an integral carrier such as a strip of material, dental tray, and/or sponge material, and thereafter applied to the teeth. In one embodiment the compositions and/or delivery systems herein are almost unnoticeable when applied to the teeth.

Then, any residual composition may be easily removed by wiping, brushing or rinsing the oral surface after a desired period of time has elapsed, or in the normal course of tooth brushing or other oral care activities.

It is not necessary to prepare the teeth before applying the present invention. For example, the user may or may not choose to brush the teeth or rinse the mouth before applying the present invention. The surfaces of the oral cavity are neither required to be dried nor to be excessively wet with saliva or water before the application. However, it is believed that adhesion to the tooth enamel surfaces will be improved if the teeth are dry prior to application.

Where the integral carrier is a strip of material, the second layer composition may be coated on the strip of material, or be applied by the user to the strip of material, or be applied by the user to the teeth and then the strip of material placed over the coated teeth. The amount of the second layer composition applied to the strip of material or teeth may depend upon the size and capacity of the strip of material, concentration of the active and the desired benefit. Generally less than 1 gram of composition is required, in one embodiment from about 0.001 grams to about 0.5 grams and in another embodiment from about 0.1 gram to about 0.4 grams of composition is used. In one embodiment the amount of composition per square centimeter (cm) of material is less than about 1 gram/cm², in another embodiment less than about 0.2 grams/cm², in another embodiment from about 0.0001 grams/cm² to about 0.1 grams/cm², and yet in another embodiment from about 0.01 grams/cm² to about 0.04 grams/cm².

The present invention may allow for a decreased frequency of application. For example, a 6% peroxide containing prior art composition that generally is used for 30 minutes twice daily for 2 weeks (e.g. for a total application time of 14 hours), may show substantially identical whitening efficacy by administering the same level of peroxide but used in accordance with the present invention, wherein the total application time is reduced to 6-10 hours. For example, when used in accordance with the present invention the same level of bleach agent may achieve equal or similar efficacy with one 30 minute application per day for 14 days or a 30 minute application twice daily for 7-10 days compared with a bleach composition of the prior art with the same bleach level.

Dental tray appliances may be used as follows. The patient or dental professional dispenses the present composition into a soft or rigid dental appliance and then the subject places the appliance over the subject's dental arch (or fits the device around his or her teeth to keep the tray in position). Generally, the recommended treatment period is in one embodiment a sufficient period of time to achieve whitening as disclosed above. At the end of the treatment period, the dental appliance is removed, cleaned with water to remove any remaining composition, and then stored until the next application.

The above-described compositions and delivery systems may be combined in a kit which comprises: 1. composition and 2. instructions for use; or comprises: 1. composition, 2. instructions for use, and 3. an integral carrier.

The compositions of this invention are useful for both human and other animals (e.g. pets, zoo, or domestic animals) applications.

### EXAMPLES

The following non-limiting examples further describe preferred embodiments within the scope of the present invention. Many variations of these examples are possible without departing from the scope of the invention.

### EXAMPLE I

Compositions of the present invention containing a bleaching agent, made by conventional processing techniques, are described below:

| Material | #1 (%W/W) | #2 (%W/W) | #3 (%W/W) | #4 (%W/W) | #5 (%W/W) |
|---|---|---|---|---|---|
| H2O2 (35% solution) | 17 | 1.43 | 17 | 17 | 17 |
| Mineral oil | 77.9 | 93.33 | 73.9 | | |
| Aerosol OT | 1 | 1 | 1 | | |
| Polysorbate 80 | | | | 1 | 1 |
| Silica | | | 4 | | |
| Water | 4.1 | 4.24 | 4.1 | 4.1 | 2.99 |
| Versagel M750² | | | | | 60 |
| Lipowax D³ | | | | | 7 |
| Thickener | | | | | 11.985 |
| EDTA | | | | 0.025 | 0.025 |
| Olive oil | | | | 77.875 | |

| | | | | | |
|---|---|---|---|---|---|
| ²Mineral oil and ethylene/propylene/styrene copolymer, and butylene/ethylene/styrene copolymer, available from Penreco. ³ Cetearyl alcohol and Ceteareth 20, available from Lipo Chemical. | | | | | |

| Material | #1 (%W/W) | #2 (%W/W) | #3 (%W/W) | #4 (%W/W) |
|---|---|---|---|---|
| H2O2 (35% solution) | 17 | 6 | 17 | 17 |
| Mineral oil | 74 | 83 | 63 | |
| Aerosol OT | 1 | 1 | 1 | |
| Polysorbate 80 | | | | 1 |
| Silica | | | 4 | |
| Water | 8 | 10 | 15 | 10 |
| Thickener | | | | |
| EDTA | | | | 0.025 |
| Olive oil | | | | 71.975 |

About 0.1-0.2g or more of the above composition may be applied directly to the teeth by any application methods disclosed herein. Alternatively the above compositions may be combined with an integral carrier such as a strip of material or tray. About 0.1-0.2g or about 1-3 g of the above composition may be combined with a strip of material or tray, respectively. In one embodiment the strip of material is a 0.013 mm thick piece of polyethylene film. The strip of material may be provided with an array of shallow pockets, typically 0.4 mm across and 0.1 mm deep. The strip of material has a flexural stiffness of about 0.6 grams/centimeter as measured on a Handle-O-Meter, model #211-300, available from Thwing-Albert Instrument Co. of Philadelphia, PA, as per test method ASTM D2923-95.

Any of the oral care compositions described above can be used with any of the integral carriers described herein.

One example of a tray is a disposable U-shaped soft foam tray, which may be individually packaged, and which may be saturated with a pre-measured quantity of the composition of the present invention. The soft foam material is generally an open celled plastic material. Such a device is commercially available from Cadco Dental Products in Oxnard, Calif. under the tradename VitalWhite™. In one embodiment these soft foam trays comprise a backing material (e.g. a closed cell plastic backing material) to minimize the elution of the bleaching agent from the device, into the oral cavity to minimize ingestion by the patient and/or irritation of the oral cavity tissues. The above examples have a Whitening Index of from about 0.5 to about 4.

## Claims

1. A bleaching composition, in the form of a water-in-oil emulsion, for whitening teeth comprising:
a) from 1% to 45%, by weight, of an aqueous phase;
b) a safe and effective amount of a bleaching agent;
c) at least 30%, by weight, of an inert, continuous, hydrophobic phase;
d) from 0.001% to 30%, by weight, of an emulsifier;
wherein the composition is an emulsion in which the inert hydrophobic phase is in predominant proportion relative to the aqueous phase.

2. The composition of Claim 1 comprising from 3% to 35% by weight of the aqueous phase.

3. The composition of Claim 1 or Claim 2 comprising from 40% to 90% by weight of the inert hydrophobic phase.

4. The composition of any preceding claim comprising from 0.1% to 20% by weight bleaching agent selected from peroxides, metal chlorites, perborates, percarbonates, peroxyacids, persulfates, hydrogen peroxide, urea peroxide, calcium peroxide, carbamide peroxide, and mixtures thereof.

5. The composition of any preceding claim wherein the inert hydrophobic phase is selected from non-toxic edible oils, saturated or unsaturated fatty alcohols, silicones, polysiloxanes, saturated aliphatic hydrocarbons, long chain triglycerides, fatty esters, and mixtures thereof.

6. The composition of any preceding claim wherein the inert hydrophobic phase is selected from mineral oil, castor oil, linseed oil, rape oil, peanut oil, soybean oil, rice bran oil, coconut oil, palm oil, safflower oil, olive oil, vegetable oils, corn oil; sesame oil; hydrogenated castor oil; partially hydrogenated soybean oil; glyceryl trioleate; glyceryl trilinoleate; an Ω-3 polyunsaturated fatty acid triglyceride containing oil; and mixtures thereof.

7. The composition of Claim 1 or 2 wherein the emulsifier has an HLB value of from 1 to 10, preferably wherein the emulsifier is a nonionic surfactant having an HLB of from 1 to 8.

8. An oral care delivery system (10) comprising:
a) an integral carrier, preferably a polyethylene strip of material (12) having a film thickness of less than 0.1 mm, and
b) a bleaching composition according to any preceding claim coated on the carrier.

9. The delivery system of Claim 8, wherein the integral carrier is a strip, further comprising an adhesive composition spatially separated from the bleaching composition.

10. The use of an oral care delivery system according to Claim 8 or Claim 9 for whitening teeth, wherein the carrier is adhered to the teeth by the bleaching composition.

## Patentansprüche

1. Bleichende Zusammensetzung in Form einer Wasser-in-Öl-Emulsion zum Aufhellen von Zähnen, umfassend:
a. zu 1 Gew.-% bis 45 Gew.-% eine wässrige Phase,
b. eine sichere und wirksame Menge eines Bleichmittels,
c. zu mindestens 30 Gew.-% eine inerte, kontinuierliche, hydrophobe Phase,
d. zu 0,001 Gew.-% bis 30 Gew.-% einen Emulgator,
wobei die Zusammensetzung eine Emulsion ist, in der die inerte hydrophobe Phase gegenüber der wässrigen Phase überwiegt.

2. Zusammensetzung nach Anspruch 1, zu 3 Gew.-% bis 35 Gew.-% die wässrige Phase umfassend.

3. Zusammensetzung nach Anspruch 1 oder Anspruch 2, zu 40 Gew.-% bis 90 Gew.-% die inerte hydrophobe Phase umfassend.

4. Zusammensetzung nach einem der vorstehenden Ansprüche, zu 0,1 Gew.-% bis 20 Gew.-% ein Bleichmittel umfassend, das ausgewählt ist aus Peroxiden, Metallchloriten, Perboraten, Percarbonaten, Peroxysäuren, Persulfaten, Wasserstoffperoxid, Harnstoffperoxid, Calciumperoxid, Carbamidperoxid und deren Mischungen.

5. Zusammensetzung nach einem der vorstehenden Ansprüche, wobei die inerte hydrophobe Phase ausgewählt ist aus nicht-toxischen, genießbaren Ölen, gesättigten oder ungesättigten Fettalkoholen, Silikonen, Polysiloxanen, gesättigten aliphatischen Kohlenwasserstoffen, langkettigen Triglyceriden, Fettsäureestern und deren Mischungen.

6. Zusammensetzung nach einem der vorstehenden Ansprüche, wobei die inerte hydrophobe Phase ausgewählt ist aus Mineralöl, Rizinusöl, Leinsamenöl, Rapsöl, Erdnussöl, Sojabohnenöl, Reiskleieöl, Kokosnussöl, Palmöl, Safloröl, Olivenöl, Pflanzenölen, Maisöl, Sesamöl, gehärtetem Rizinusöl, teilgehärtetem Sojabohnenöl, Glyceryltrioleat, Glyceryltrilinoleat, einem mehrfach ungesättigtes Ω-3-Fettsäuretriglycerid enthaltendem Öl und Mischungen davon.

7. Zusammensetzung nach Anspruch 1 oder 2, wobei der Emulgator einen HLB-Wert von 1 bis 10 aufweist, wobei der Emulgator vorzugsweise ein nichtionisches Tensid mit einem HLB von 1 bis 8 ist.

8. Mundpflegeabgabesystem (10), umfassend:
a) einen integralen Träger, vorzugsweise einen Polyethylenstreifen aus Material (12) mit einer Schichtdicke von weniger als 0,1 mm, und
b) eine bleichende Zusammensetzung nach einem der vorstehenden Ansprüche, die auf den Träger aufgebracht ist.

9. Abgabesystem nach Anspruch 8, wobei der integrale Träger ein Streifen ist, der ferner eine Haftmittel-Zusammensetzung aufweist, die räumlich von der bleichenden Zusammensetzung getrennt ist.

10. Verwendung eines Mundpflege-Abgabesystems nach Anspruch 8 oder Anspruch 9 zum Aufhellen von Zähnen, wobei der Träger durch die bleichende Zusammensetzung an den Zähnen haftet.

## Revendications

1. Composition de blanchiment, sous la forme d'une émulsion eau dans huile, pour le blanchiment des dents, comprenant :
a de 1 % à 45 %, en poids, d'une phase aqueuse ;
b. une quantité efficace et sans danger d'un agent de blanchiment ;
c. au moins 30 %, en poids, d'une phase hydrophobe, continue et inerte ;
d. de 0,001 % à 30 %, en poids, d'un émulsifiant ;
où la composition est une émulsion dans laquelle la phase hydrophobe inerte est en proportion prédominante par rapport à la phase aqueuse.

2. Composition selon la revendication 1 comprenant de 3 % à 35 % en poids de la phase aqueuse.

3. Composition selon la revendication 1 ou la revendication 2 comprenant de 40 % à 90 % en poids de la phase hydrophobe inerte.

4. Composition selon l'une quelconque des revendications précédentes comprenant de 0,1 % à 20 % en poids d'agent de blanchiment choisi parmi des peroxydes, chlorites métalliques, perborates, percarbonates, peroxyacides, persulfates, peroxyde d'hydrogène, peroxyde d'urée, peroxyde de calcium, peroxyde de carbamide et leurs mélanges.

5. Composition selon l'une quelconque des revendications précédentes dans laquelle la phase hydrophobe inerte est choisie parmi des huiles alimentaires non toxiques, des alcools gras saturés ou insaturés, des silicones, des polysiloxanes, des hydrocarbures aliphatiques saturés, des triglycérides à chaîne longue, des esters gras et leurs mélanges.

6. Composition selon l'une quelconque des revendications précédentes dans laquelle la phase hydrophobe inerte est choisie parmi de l'huile minérale, de l'huile de ricin, de l'huile de lin, de l'huile de colza, de l'huile d'arachide, de l'huile de soja, de l'huile de riz, de l'huile de noix de coco, de l'huile de palme, de l'huile de carthame, de l'huile d'olive, des huiles de légumes, de l'huile de maïs ; de l'huile de sésame ; de l'huile de ricin hydrogénée ; de l'huile de soja partiellement hydrogénée ; du trioléate de glycéryle ; du trilinoléate de glycéryle ; une huile contenant des triglycérides d'acide gras polyinsaturés Ω-3 ; et leurs mélanges.

7. Composition selon la revendication 1 ou 2 dans laquelle l'émulsifiant a une valeur HLB de 1 à 10, de préférence dans laquelle l'émulsifiant est un tensioactif non ionique ayant un HLB de 1 à 8.

8. Système d'administration de soin dentaire (10) comprenant :
a) un véhicule intégral, de préférence une bande polyéthylène de matière (12) ayant une épaisseur de film inférieure à 0,10 mm, et
b) une composition de blanchiment selon l'une quelconque des revendications précédentes enduite sur le véhicule.

9. Système d'administration selon la revendication 8, dans laquelle le véhicule intégral est une bande, comprenant en outre une composition adhésive séparée spatialement de la composition de blanchiment.

10. Utilisation d'un système d'administration de soin dentaire selon la revendication 8 ou la revendication 9 afin de blanchir les dents, où l'on fait adhérer le véhicule aux dents par la composition de blanchiment.
